# EUROPEAN PATENT APPLICATION

(11) **EP 1 574 438 A1**
(43) Date of publication of application: **14.09.2005**
(21) Application number: 04010378.0
(22) Date of filing: 30.04.2004
(51) Int. Cl.: B65D 1/02

(54) **Distribution system of pet bottle for drinking water and beverage**

(30) Priority: 10.03.2004 US 796339
(71) Applicant: Gohsho Company, Ltd., Yamanashi (JP)
(72) Inventor: Higuchi, Mitsuo, Nakakoma-gun, Yamanashi (JP)
(74) Representative: Kirschner, Klaus Dieter

(57) **Abstract**

A system for a PET bottle body for drinking water and beverage using a container in a bellows shape, in which a series of steps for housing and transporting a large quantity of PET bottle bodies at one time from a manufacturing step, to a charging factory of content and after the PET bottle bodies are emptied are performed accurately, reliably and easily is proposed. When the PET bottle body charged with mineral water or juice are purchased and the mineral water or juice is drunken, the volume and height of the PET bottle body can be reduced corresponding to the emptied amount, and after the PET bottle body is emptied, the PET bottle body is compressed in the vertical direction by being crushed, so that the PET bottle body can be discarded in a state in which its volume is much smaller than the predetermined volume and its height is very low.

## Description

### BACKGROUND OF THE INVENTION

### Field of the Invention

The present invention relates to a distribution system for transporting from a manufacturing process to a reclaiming plant for disposal, recycling and the like, containers for juice and mineral water mainly formed by using a blow molding method such as stretch blow and indexing blow, which is generally called a PET (polyethylene terephthalate) resin blow or the like.

### Description of the Related Art

The amount of production of synthetic resin bottles has been tremendous in recent years.

However, such bottles have the largest disadvantage that when the juice or mineral water inside the PET bottle bodies is drunken off and the PET bottle bodies are discarded, they remain in the shape before they are not emptied, when they are discarded into so-called garbage boxes, the garbage boxes become quickly full as if air were discarded into the garbage boxes, and therefore they are ultimately discarded on the streets to worsen the living environments. In addition, the recovering cost of the emptied PET bottle bodies and the labor cost for cleaning become a large burden on the public works.

This kind of synthetic resin bottles can be manufactured by the easy means such as blow molding, but the strength thereof cannot be said sufficient enough to correspond to automobiles and the other transportation means accompanied by vigorous vibrations, and display at the stores in which they are stacked on one another.

Further, this makes the ratio of the transportation cost of the products considerably high because the PET bottle bodies are so bulky that when the container bottles are transported to the companies for charging juice and mineral water therein from the manufacturers of the container bottles, it seems as if air were transported.

Consequently, the inventor of the present application has already provided a liquid container which is designed to be contracted simply when the liquid container as a waste is recovered.

This is made of a comparatively soft synthetic resin, and the peripheral wall of a liquid PET bottle body with a mouth section on the upper end is formed into a bellows shape (refer to Patent Document 1).

In addition to the above, the inventor has proposed that the PET bottle body is formed into the shape which is substantially contracted in it volume by applying a load in the vertical direction and / or the twisting direction of the PET bottle body, and the invention of the means to keep the contracted shape and the PET bottle body.

As a result, both of them can sufficiently attain the above described object, namely, to reduce the height and the container more when the PET bottle body is crushed and keep the reduced state.

The means for manufacturing this kind of bellows-shaped container is invented as described above, but there is virtually no invention of the system for transporting it to the plants for disposal or recycling.
[Patent Document 1] Japanese Patent Laid-Open No. 2001-213418 (Abstract)
[Patent Document 2] Japanese Patent Laid-Open No. 2002-068156 (Abstract)

### SUMMARY OF THE INVENTION

Hence, an object of the present invention is to make it possible to manufacture a PET bottle easily, which is reduced in its vertical width (height) substantially as compared when the content is charged in the PET bottle body, upon manufacturing the PET bottle body in the above described series of the system.

Another object of the present invention is to design the above described compressed PET bottle body to be able to keep the state at a normal temperature.

Still another object of the present invention is to include means capable of transport the PET bottle bodies remaining in the compressed state from a manufacturing process (factory) to a factory for charging the content.

Yet another object of the present invention is to design the PET bottle body to be able to have the equal volume as the original volume again by applying inner pressure or the like in the above described charging factory.

Another object of the present invention is to design the PET bottle body to be contractible in the volume corresponding to the emptied amount, when a purchaser of this product is drinking the content, or finishes drinking it, after the PET bottle body charged with the above-described content is transported to the retailer in the charged state.

Still another object of the present invention is to design the PET bottle which becomes "empty" after the beverage or water is drunken off to be contractible into the volume (height) of 1/2 to 1/10 of the original volume, and increase the volume, namely, the processing amount which can be handled at one time dramatically as compared with the prior art when it is housed in the garbage box and is transported for disposal and reuse.

Generally, an object of the present invention is to propose a system in which a series of processes of manufacturing the PET bottles for drinking water and beverage using the above bellows-shaped containers, charging contents therein in a charging factory, and housing and transporting a large number of the PET bottle bodies at one time after they are emptied are carried out accurately, reliably, easily and smoothly.

Hence, a characteristic of the present invention is, in a process of an operation of manufacturing the PET bottle bodies for mineral water, juice and the like to disposal of the PET bottle bodies after the mineral water, the juice and the like are drunken up, having a step of forming a PET bottle body, in which a part or all of the PET bottle body except for vertical width of a mouth section of an upper end portion is in a bellows shape in a horizontal direction, into a predetermined volume and height.

Another characteristic of the present invention is, in the above described process, having a step of performing a forming work to compress this formed PET bottle body in a vertical direction to be much smaller in volume than the aforesaid predetermined volume and very low in height, and to make it possible to keep the compression state at a normal temperature.

Still another characteristic of the present invention is, in the above described process, having a step of transporting the compressed PET bottle bodies to a charging factory of mineral water, juice and the like while keeping the state.

Yet another characteristic of the present invention is, in the above described process, having a step of expanding the PET bottle body again to the volume and height at the time of the above-described forming when air is forced into the PET bottle body, or the PET bottle body is sterilized or cleaned, or on the occasion of an operation of charging the PET bottle body with mineral water, juice and the like in this charging factory.

Another characteristic of the present invention is, in the above described process, having a step of transporting the PET bottle bodies with the predetermined volume and height, which are charged with the mineral water, juice and the like, to wholesalers or retailers.

A general characteristic of the present invention is a distribution system of the PET bottles for drinking water and beverage, in which when the PET bottle body charged with the mineral water, juice or the like is purchased and the mineral water or juice is drunken, the volume and height of the PET bottle body can be reduced corresponding to an emptied amount, and after the mineral water or juice is finished, the PET bottle body is compressed in a vertical direction by being crushed, so that the PET bottle body can be discarded in a state with a much smaller volume than the aforesaid predetermined volume and very low height.

The entire shape of the PET bottle body in the present invention is properly selected from circular cylinders including an elliptic cylinder, square columns including a rectangular column, circular cones and pyramids including those with heads cut, a Hourglass dram-shape, and a barrel shape. The shape in a horizontal section of the above described PET bottle body is selected from round shapes including an ellipse, and square shapes including a rectangular shape. The present invention can be applied to the manufacturing means of such a container as the above described PET bottle around the outer periphery of which a label with a guide and trade name of the container can be wound. All of these are considered within the technical scope of the present invention.

The present invention has the other excellent object, characteristics and operational effects, and these will become apparent in the following explanation of the embodiment.

### BRIEF DESCRIPTION OF THE DRAWINGS

FIG. 1 is an explanatory view of a distribution process from production of a PET bottle body to disposal;
FIG. 2 is a front view of an entire PET bottle body in one embodiment;
FIG. 3 is a front view of the same PET bottle body when it is crushed to be small;
FIG. 4 is an enlarged sectional view of a bellows shape portion of the PET bottle body;
FIG. 5 is an enlarged sectional view of a shoulder portion of the same PET bottle body;
FIG. 6 is a sectional explanatory view of a die body;
FIG. 7 is a sectional explanatory view a compression forming mechanism, and shows the state before the PET bottle body is crushed;

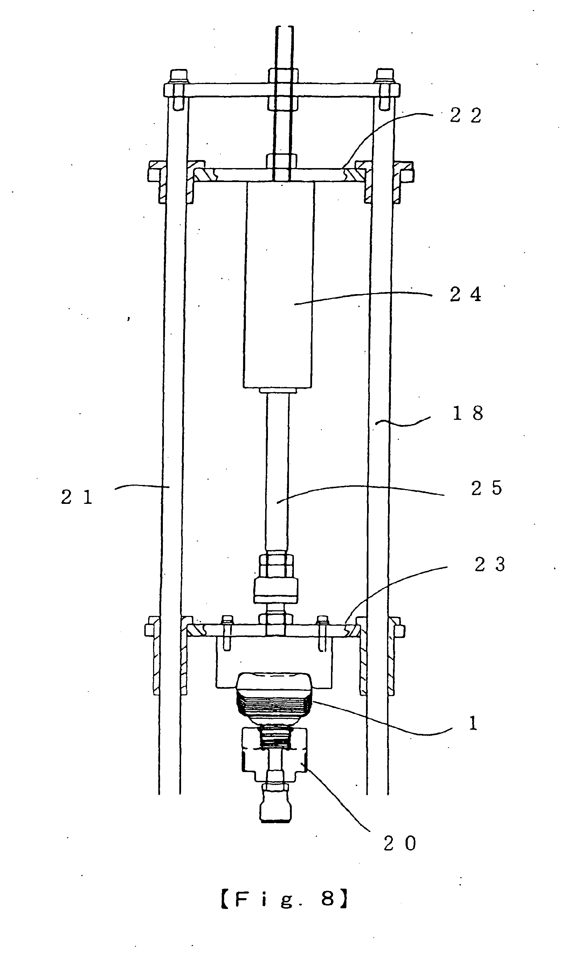

FIG. 8 is a sectional explanatory view showing the state of the PET bottle body after it is crushed;
FIG. 9 is a block diagram of piping of the compression forming mechanism;
FIG. 10 is a sectional explanatory view of a state in which the PET bottle body is crushed;
FIG. 11 is a sectional explanatory view of the bellows-shaped portion of the same PET bottle body; and
FIG. 12 is a sectional explanatory view of a bottom section of the same PET bottle body.

### [Description of Symbols]

- (A): Height
- (A)': Height
- (L'): Height
- (L): Height
- A: Body section
- B: Bottom section
- C: Shoulder at upper potion
- d1: Outer diameter
- d2: Outer diameter
- d3: Outer diameter
- h1: Upper portion of wall of first step 5
- h2: Lower portion of wall of first step 5
- 1: PET bottle body
- 2: Bellows shape
- 3: Mouth section of drinking
- 4: Back section
- 5: First step of bellows shape
- 6: Second step of bellows shape
- 7: Third step of bellows shape
- 8: Fourth step of bellows shape
- 9: Bent shape
- 10: Protruded arc shape
- 11: Linear shape
- 12: Peak
- 13: Valley
- 14: Upper surface
- 15: Lower surface
- 16: Die body
- 17: Preform
- 18: Forming mechanism
- 19: Horizontal stand
- 20: Chuck mechanism
- 21: Support pillar
- 22: support rod
- 23: Support rod
- 24: Cylinder
- 25: Piston shaft
- 26: Pressing member
- 27: Pressure source
- 28: Pipe
- 29: Three-directional valve
- 30: Proper shut-off valve
- 31: Piston board
- 32: Transportation
- 33: Cleaning
- 34: Sterilizing operation
- 35: Charging operation
- 36: Store
- 37: Half-finished
- 38: About 1/4 to 1/10
- I: Sectional explanatory view of die body
- II: Sectional explanatory view of compression forming mechanism, showing state before container body is crushed
- III: Sectional explanatory view showing state of container body after it is crushed
- IV: Sectional explanatory view of state in which container body is crushed
- V: Transportation
- VI: Charging factory
- VII: Transportation
- VIII: Store
- IX: For Drink
- X: Garbage box

### DETAILED DESCRIPTION OF THE PREFERRED EMBODIMENTS

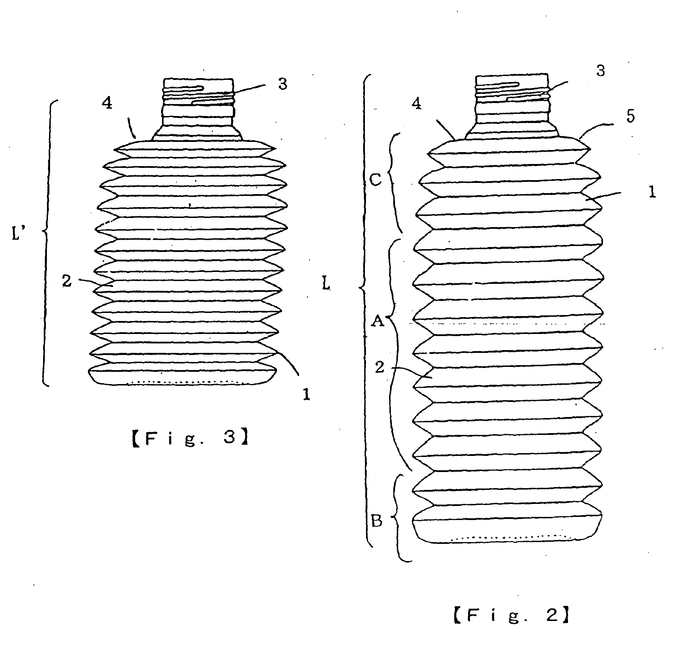

Reference numeral (1) in the drawings denotes a PET bottle body obtained by a manufacturing method of the present invention, FIG. 2 shows a state in which the PET bottle body (1) is removed from a die body (16) which molds the PET bottle body (1), namely, a volume height (L) thereof is equivalent to that when a content is charged into the PET bottle body (1). FIG. 3 shows a state after a forming work in a forming work mechanism (18) for compressing the PET bottle body (1) in a vertical direction, namely, the volume height (L') is in a state of zero, and the above described volume height can be normally reduced to about 1/2 to 1/10 of the volume height (L).

On explaining an embodiment of the present invention based on the drawings, the characteristics of the present invention can be easily understood if the structure and the operational effects of the PET bottle body (1) itself are understood, and therefore the explanation thereof will be made.
Namely, the PET bottle body (1) shown in FIG. 2 is in the state in which it is removed from the manufacturing die, and an outer periphery and an inner periphery in its height (L) [length] direction except for a mouth section at an upper portion are in a bellows shape (2). In this case, the height (L) of the PET bottle body (1) is that of the volume of 500 m/ml at the time when charged with content.

More specifically, the PET bottle body (1) in FIG. 2 is 195 mm high with a maximum diameter of 71.5 mm, and a volume of 500 m/ml, and has a shape having a wall in the bellows shape (2) with 12 steps. Reference numeral (3) in the drawings is the mouth section for drinking, and reference numeral (4) is a back section from this mouth section to the first step (5) of the bellows shape.

Hence, an outer diameter of the first step (5) of the bellows shape is set at 67.9 mm, an outer diameter of a second step (6) of the bellow shape is set at 69.7 mm, and a space between the first step (5) of the bellows shape and the second step (6) of the bellows shape is set at 14 mm, in this embodiment.
Similarly to this, an outer diameter of a third step (7) of the bellows shape is set at 71.5 mm, and a diameter of a portion between the second step (6) of the bellows shape and the third step (7) of the bellows shape is set at 54.5 mm.

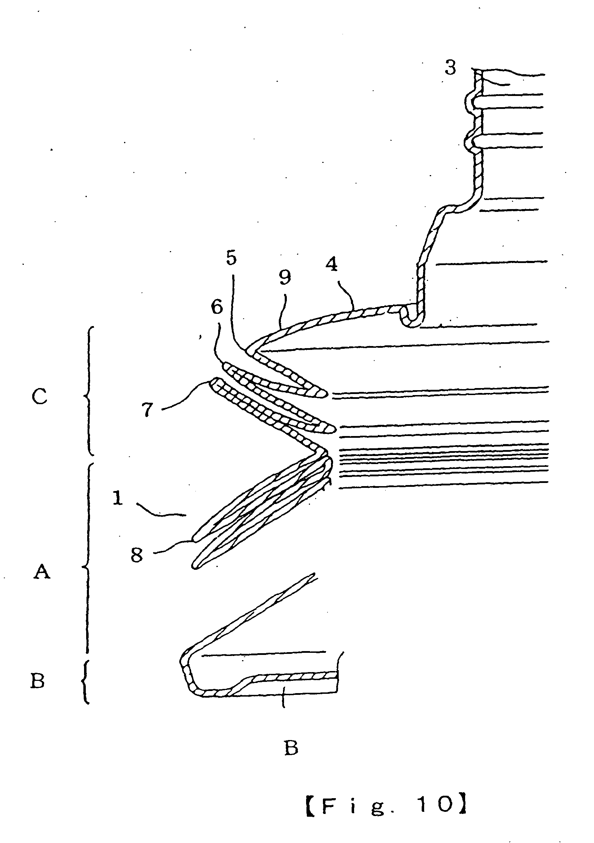

Further, an outer diameter of a fourth step (8) of the bellows shape in FIG. 10 is 71.5 mm which is the same as the outer diameter of the above described third step (7) of the bellows shape being the maximum outer diameter of the above described PET bottle body (1), and a diameter of a portion between the third step (7) of the bellows shape and the fourth step (8) of the bellows shape is also set at 54.5 mm as described above.

Outer diameters of the following fifth step to twelfth step of the bellows shape are the same as the outer diameter of the fourth step (8) of the bellows shape, and each inner diameter between them is the same as that between the above described third step (7) of the bellows shape and the fourth step (8) of the bellows shape.

In this case, each of the above described wall is in such a shape as an unidentified flying object (UFO) called Adamski type, and therefore it is horizontally divided into two of an upper and lower parts.

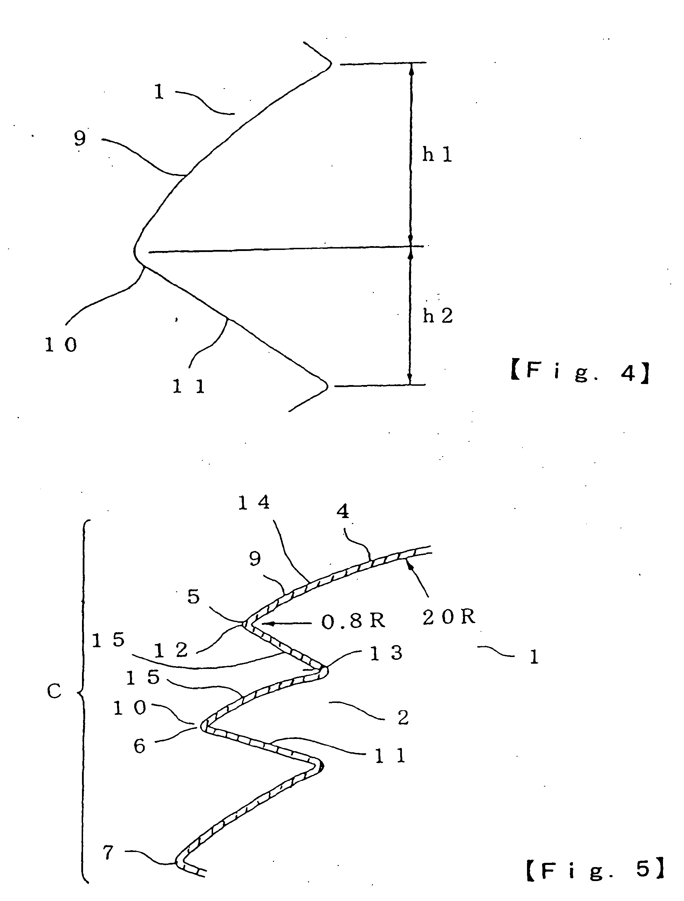

At this time, the upper height (h1) and the lower height (h2) of the wall of the above described first step (5) are each set to be 2.75 mm in FIG. 4. In contrast to this, that of the wall of the second step (6) is 8.5 mm in the upper part and 6 mm in the lower part, and that of the wall of the third step (7) is 8.5 mm in the upper part and 6.5 mm in the lower part.

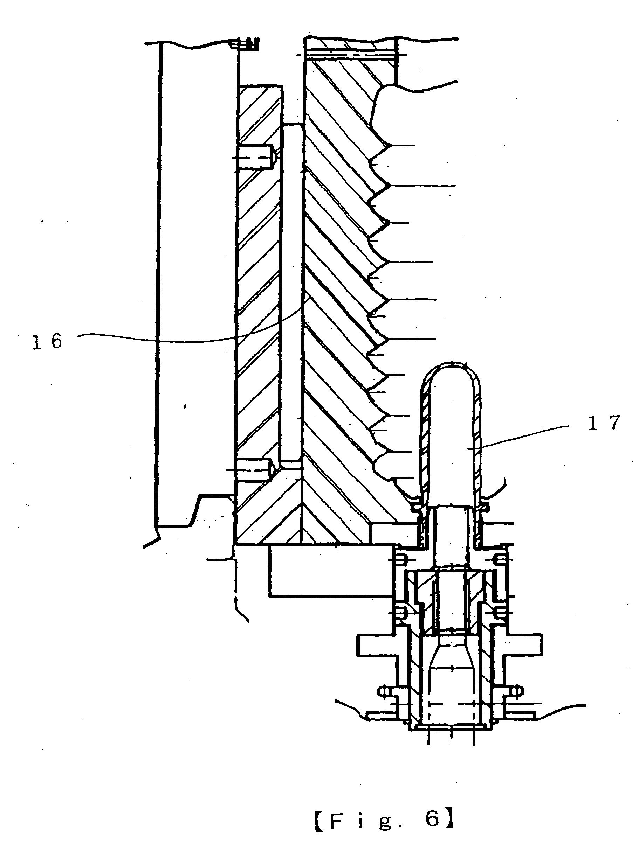

FIG. 5 is more concrete example of the dimensions of the walls of the above described first step (5) to the third step (7), and it is the important point that the shape of an upper surface (14) with large width in a shape of the bead of the Japanese abacus is a curved shape (9) with 20 mmR protruded outward in this embodiment, while a lower surface (15) is in a linear shape (11) via a protruded arc shape (10) with 0.8 mmR.

Explaining the construction of this embodiment in combination with its operation, the PET bottle body (1) in the state of the above described FIG. 2 is generally divided into three that are a body section (A) including a central part and a lower part, a bottom section (B) and a shoulder section (C) at an upper part as shown in FIG. 10, and in the sections (A) and (C), each diameter is constituted of two diameters that are a large diameter and a small diameter in the comparison of each of them. Consequently, it keeps a stable vertical state.

Namely, the body section (A) including the above-described central part and the lower part is formed of the wall with the same inner and outer diameters, and the shoulder part (C) is bent in a protruded shape with a gentle inclination in the direction of the mouth section (3) in the external appearance. Especially in this shoulder section (C), in the area from a central portion to the mouth section (3), the outer diameters of a peak (12) and a valley (13) forming the wall are gradually decreased in the direction of the mouth section (3), and the difference in the inclination angle is made larger than in the body section A. As a result, the inner diameter and inclined surface of the peak (12) and the valley (13) forming the wall in the state in which this central portion is extended can keep the state in which the height is reduced with the strength and elastic force of the material even if drinking operation is stopped even while the content is drunken, and an empty portion is crushed by compressing the PET bottle body (1) in the vertical direction.

The shoulder section (C) and the body section (A) of the PET bottle body (1) press the wall of the shoulder section C which is to be in a reduced state with their restoring force, and an end surface of the valley (13) compresses a valley end surface of the wall located adjacently thereto in the vertical relation.

In the state seen from the vertex of the peak (12) of the wall at this point of time, it seems that the valleys (13) at both sides are pressed near to the vertex of this peak (12). In this case, since the upper surface (14) of the wall has smaller inclination with respect to the lower surface (15), the compression component force at the side of the gentle inclination of the upper surface (14) is larger than the compression component force at the side of the sharp inclination of the lower surface (15), and therefore the valley (13) at the side of the sharp inclination moves to the vertex of the peak (12) portion.

It is considered that two large changes occur to the PET bottle body (1) at this time.
Namely, the first is that the inner diameter of the peak (12) portion constructing the above described wall is increased by the extending pressure, or the inner diameter of the valley (13) is decreased by the compression pressure. The second is that the sharp inclination surface of the lower surface (15) constructing the wall is bent.

Subsequently, when the sharp inclination side passes directly under the peak (12) portion, slips inside the gentle inclination side of the upper surface (14) as seen in the drawing, and the height of the PET bottle body (1) is moved into a shortened state, the force to restore the inner diameter of the peak (12) and the inner diameter of the valley (13) works, or the lower surface (15) which is brought into the bent state by the above described operation is restored into the extended state to be in a stable state.
Accordingly, even if the compressing force is not always applied, the shortened state can be kept.

The valley (13) with the small inner diameter receives the compression pressure, then the inner diameter thereof becomes further smaller, and pressure stress occurs.
When the compression pressure does not exist, the stress works to be released, and the valley is restored into the extension state.
Consequently, the PET bottle body (1) keeps shortened state at the atmospheric pressure, and when the compression stress does not occur due to the difference of the inner diameter of the valley (13), the shortened state is kept.

As a result of the repeated experiments, the height of the PET bottle body (1) can be made 1/2 to 1/10 or less of the original height, and even if this is discarded into a garbage box or the like, the space occupied by it can be reduced substantially.

Next, an example of a die suitable for mass production of the PET bottle body (1) of the present invention will be described.

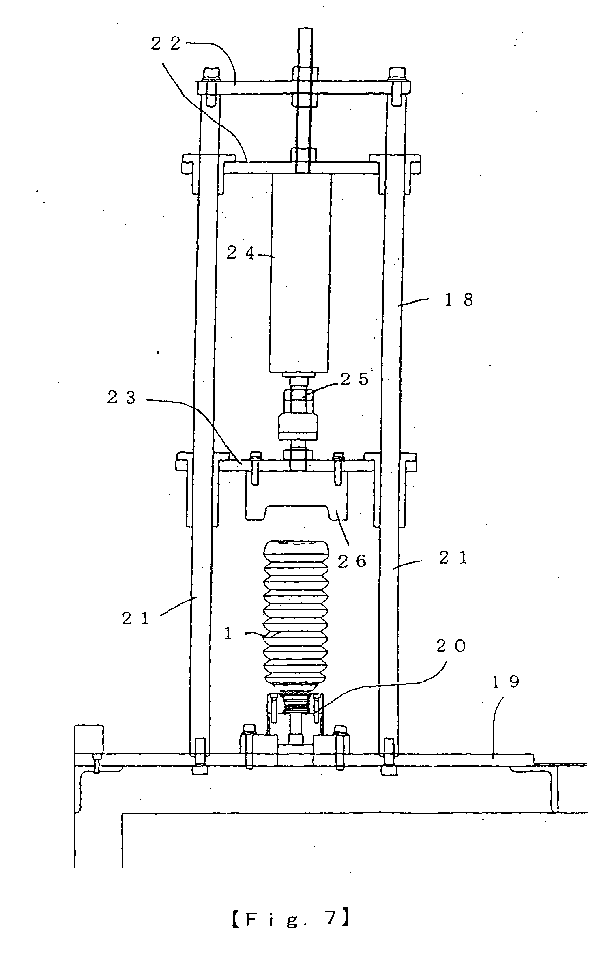

Namely, in FIG. 6, reference numeral (16) denotes a two-split die body for producing the cylindrical PET bottle body (1) in FIG. 2, and a preform (17) can be inserted from its bottom surface.

Explaining the process of producing the PET bottle body (1) by using the die body (16) in this embodiment, the die body (16) is initially in an opened state.

In this state, the preform (17) is automatically inserted into the die body (16) from below. After heated uniformly, high pressure air of about 40 kg is blown into the preform (17) to expand the preform (17) to form the PET bottle body (1).
The bet bottle body (1) thus obtained has the equal volume height as when this container is charged with a content such as juice.

In FIG. 7, reference numeral (18) denotes a mechanism to perform compression forming for the PET bottle body (1), and reference numeral (19) denotes a horizontal stand. A chuck mechanism (20) constructed properly exits at a central part of the stand (19) though not shown in detail, and the mouth section (3) of the above described PET bottle body (1) is chucked by the chuck mechanism 20 so that the PET bottle body can be reliably held in the inversed vertical state.

Reference numeral (21) denotes a pair of support pillars fixed upright at both sides of the above described chuck mechanism 20, and upper and lower support rods (22) and (23) are laid across the respective support pillars at a middle portion and an upper end portion. Reference numeral (24) denotes a cylinder fixed inside both the support rods (22) and (23) in the vertical state, and a piston shaft (25) penetrates through the lower support rod (23) and has a pressing member (26) fitted onto the bottom section (B) of the above described PET bottle body (1) a little and pressing the PET bottle body (1) in the direction of the mouth section (3) from the bottom section (B) at a tail end.

The fluid supplied to this cylinder (24) may be air pressure, hydraulic pressure and the other proper fluid. The supply means is selected from the known means.

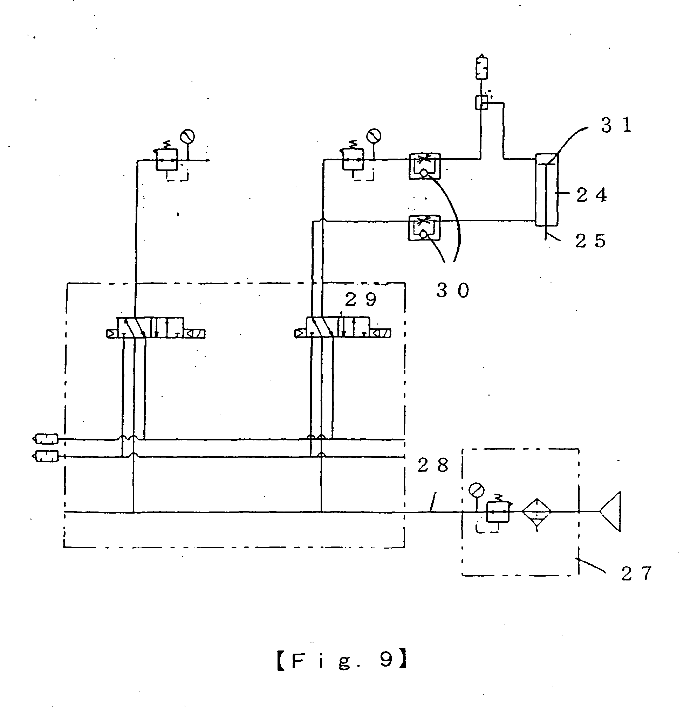

For example, in FIG. 9, reference numeral (27) is a pressure source, and its output power connects to upper and lower portions of a piston board (31) in the cylinder (24) via a pipe (28), a three-directional valve (29), and a proper shut-off valve (30).

Here, the PET bottle body (1) taken out of the above described die body (16) is set at the chuck mechanism (20) of the compression forming mechanism (18).

Next, proper fluid pressure is sent to the upper portion of the piston board (31) of the cylinder (24) via the pipe (28) from the pressure source (27). As a result, the piston board (31) and the piston shaft (25) are lowered and the pressing member (26) at the tail end abuts to the bottom section (B) of the PET bottle body (1) to compress the PET bottle body (1).

The PET bottle body (1) of the volume (small height) thus obtained has the height reduced to 1/2 to 1/10 of the original state.

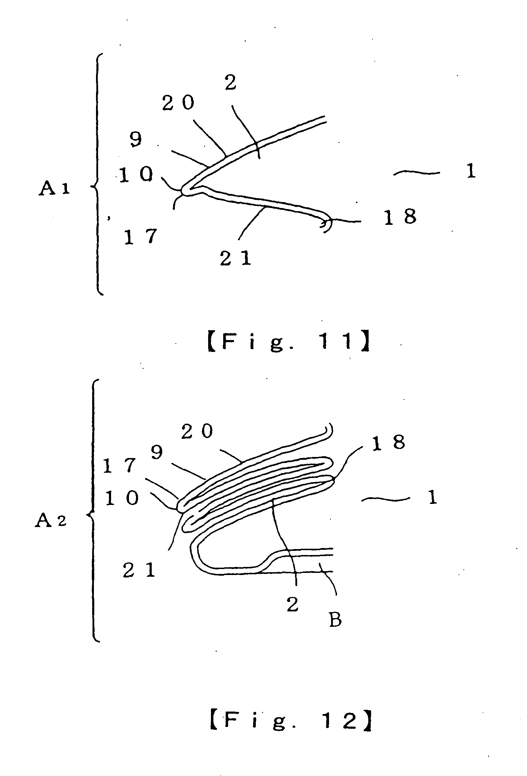

Namely, the body section (A) including the upper part and the lower part of the PET bottle (1) in this state is in the contracted state with the shoulder section (C) and the body section (A) being compressed as shown in FIG. 10 to FIG. 12. More specifically, from the state of the PET bottle body (1) taken out of the die body (16) shown in the above described FIGS. 4 and 5, the wall of the shoulder section (C) is pressed, and the end surface of the valley (13) compresses the valley end surface of the wall adjacently located in the vertical relationship.

In the state seen from the vertex of the peak (12) at this point of time, it seems the valleys (13) at both sides are pressed to the vertex of the peak (12). In this case, since the compression component force of the upper surface (14) of the wall is larger than the lower surface (15) at the sharp inclination side, the valley (13) at the sharp inclination side moves to the vertex of the peak (12) portion, which is described above. In the crushed PET bottle body (1), a blastmere shape and the curved shape (9) forming the inclined part at the upper portion of the wall of the back section (4), and the inclined portion of the lower part of the wall of the body section (A) are so-called "balanced", and therefore the PET bottle body (1) can keep the crushed state without putting on the cap.

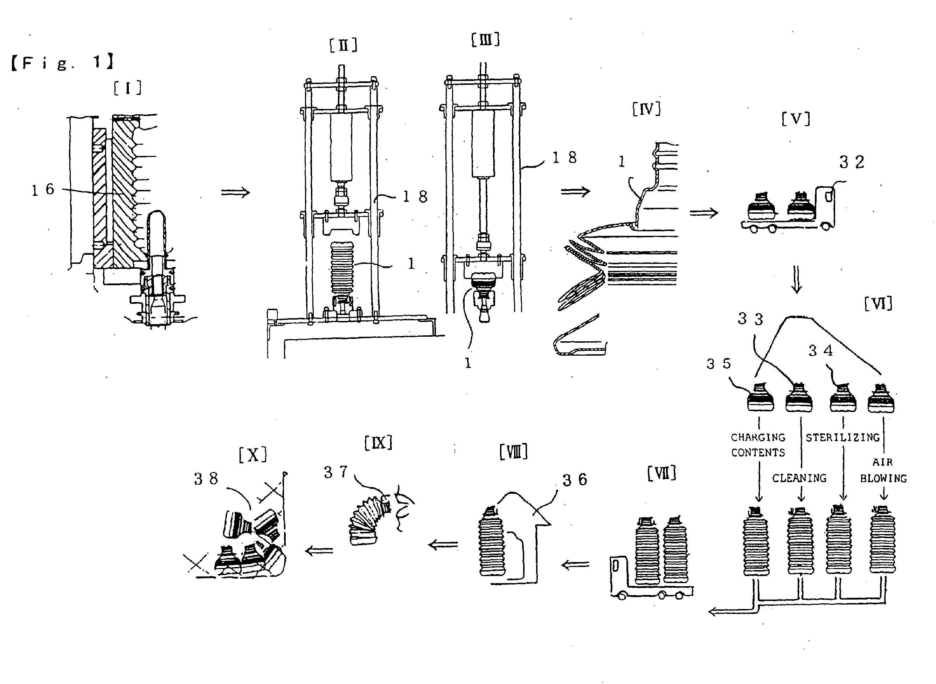

In FIG. 1, the distribution process from the production of the PET bottle of the present invention to the disposal of the PET bottle is shown.

Namely, [I] in the drawing is the manufacturing means of the PET bottle body (1) of the present invention as described above, and the PET bottle body (1) is produced by the die body (16). In this case, the volume (height) is (L), which is the same as when the PET bottle is charged with the filler. Next, the PET bottle body (1) is transferred to the compression forming mechanism 18 which is usually placed in the same factory, and the height of the PET bottle body (1) in [II] in the drawing before execution of the work is compressed in the vertical direction as shown in FIG. [III], whereby the container (height) can be sharply reduced as in FIG. [IV] as described above. Upon transportation (32) of this, the transporting amount of the PET bottles can be dramatically increased as compared with the prior art, and the reduced state is kept during the transportation (32) is as described above.

Next, the PET bottle body (1) in the contracted state is carried to the factory [VI] for charging the PET bottle body (1) with content such as juice or mineral water.

Though detailed illustration in the drawing or the detailed explanation is omitted, when the operation of cleaning (33) of the inside of the PET bottle body (1), and the operation of sterilization (34) after the cleaning or at the same time as the cleaning are performed in many cases, and in the case in which these operation are already done in the separate operation, when air is simply forced into the PET bottle body (1), or when the content itself is charged (35) into the PET bottle body (in the rare case), though these operations differ depending on the environment of the charging factory, the kinds of the contents to be charged, and the legal regulations of the country, and the cap and label are put on the PET bottle body (1) in its original state and the PET bottle is transported [VII].

[VIII] in the drawing shows the PET bottle body (1) arranged in the store. Since a purchaser of this can bend the PET bottle body 1 in the height direction each time the purchaser drinks the content in it, the purchaser can drink juice or the like without leakage even while lying on a beach, for example. Even during drinking (37), the purchaser can press the empty portion of the PET bottle body (1) to shorten the container (height), and by doing so, the purchaser can put even, for example, the half-finished PET bottle body (1) into a bag or the like easily and carry it.

Further, after the purchaser drinks up all the content, the purchaser crushes the empty PET bottle body (1) from the upper or lower direction or both upper and lower directions and reduces the volume to about 1/2 to 1/10 (38) with respect to the original volume and discards it into the garbage box [X] and the like, and thereby the amount of the PET bottle bodies which is housed can be increased as much as possible, and upon the recovering operation, the amount of the PET bottle bodies which is recovered can be dramatically increased.

Accordingly, the amount of so-called garbage including the crushed PET bottle bodies or the PET bottle bodies which is transported to the disposal factory or the recycling factory can be increased tremendously as compared with that of the prior art.

## Claims

1. A distribution system for PET bottles for drinking water and beverage in a process of an operation of manufacturing the PET bottle bodies for mineral water, juice and the like to disposal of the PET bottle bodies after the mineral water, the juice and the like are drunken up, comprising:
(1) a step of forming a PET bottle body, in which a part or all of the PET bottle body except for vertical width of a mouth section of an upper end portion is in a bellows shape in a horizontal direction, into a predetermined volume and height;
(2) a step of performing a forming work to compress this formed PET bottle body in a vertical direction to be much smaller in volume than said predetermined volume and very low in height, and to make it possible to keep the compression state at a normal temperature;
(3) a step of transporting the compressed PET bottle bodies to a charging factory of mineral water, juice and the like while keeping the state;
(4) a step of expanding the PET bottle body again to the volume and height at the time of the above-described forming when air is forced into the PET bottle body, or the PET bottle body is sterilized or cleaned, or on the occasion of an operation of charging the PET bottle body with mineral water, juice and the like in the charging factory; and
(5) a step of transporting the PET bottle bodies with the predetermined volume and height, which are charged with the mineral water, juice and the like, to wholesalers or retailers,
wherein when the PET bottle body charge with the mineral water, juice or the like is purchased and the mineral water or juice is drunken, the volume and height of the PET bottle body can be reduced corresponding to an emptied amount, and after the mineral water, juice or the like is finished, the PET bottle body is compressed in a vertical direction by being crushed, so that the PET bottle can be discarded in a state with a much smaller volume than said predetermined volume and very low height.
